# EUROPEAN PATENT SPECIFICATION

(11) **EP 2 346 458 B1**
(45) Date of publication and mention of the grant of the patent: **20.11.2013**
(21) Application number: 09760629.7
(22) Date of filing: 13.10.2009
(51) Int. Cl.: A61F 13/00, A61F 13/08, A47G 25/90

(54) **IMPROVED AUXILIARY DEVICE FOR WEARING A THERAPEUTIC ELASTIC STOCKING**
VERBESSERTE HILFSVORRICHTUNG ZUM TRAGEN EINES THERAPEUTISCHEN ELASTISCHEN STRUMPFES
DISPOSITIF AUXILIAIRE AMÉLIORÉ POUR PORT D'UN BAS ÉLASTIQUE THÉRAPEUTIQUE

(30) Priority: 15.10.2008 IT RM20080154 U
(43) Date of publication of application: 27.07.2011
(73) Proprietor: Varimed S.r.l., 95123 Catania (IT)
(72) Inventor: MARINO, Roberto, I-95123 Catania (CT) (IT)
(74) Representative: Tiburzi, Andrea
(86) International application number: PCT/IT2009/000461
(87) International publication number: WO 2010/044119

(56) References cited:
- WO-A1-99/44548
- FR-A1- 2 775 431
- GB-A- 2 316 600
- US-A- 5 356 057

## Description

The present invention relates to an auxiliary device for wearing therapeutic elastic stocking.

More specifically, the invention relates to a device permitting a quick and intuitive wearing of a therapeutic stocking on a limb, aiding users having some motive problems.

As it is well known, after surgical interventions of for different kind of pathologies, elastic stockings are often used, mainly comprised of synthetic fibres, said stocking having high retention properties, suitable to be applied of the concerned limbs, such as a forearm, a foot or a leg.

Typically, these stockings are used in post-surgical convalescences, such as post-surgical rehabilitation of knee arthroprosthesis, or in the vascular field, such as deep venous thrombosis, since they can reduce the risk of post-thrombosis complications.

A problem connected with elastic stockings is that they are usually difficult to be put on just because of their high retention capabilities. This problem is particularly important for elderly patients, mainly for their reduced mobility.

Auxiliary devices are available on the market since many times easing the user in wearing said retention elastic stockings on the interested limb.

The Applicant has already suggested in the Italian Patent n° 1379018, an auxiliary device comprised of natural fibre slippery tissue, and made up of two pieces of tissue partially coupled along their perimetral edge, so as to realise a kind of "slippers". Said device permits easily putting on the elastic stocking.

Said device particularly comprises slots on one of its ends, and through which a band passes that, when the stocking is put on, can be pulled making the tissue pieces folding and sliding on themselves, thus easing withdrawal of the same device from said stocking when it is put on.

Problem of the above device is that, being the band movable with respect to the stocking and having the same stocking retention properties, the latter drags band and makes the same skippering along the tissue pieces of the device. This implies that the band sometimes cannot be easily grasped after that the stocking has been removed. This is particularly inconvenient for those with reduced motion capability as well for elderly people or for those that undergo to an important surgical intervention to the limbs.

The relevant prior art includes the patent applications W02008035973 A1, US5356057 A, FR2775431 A1, GB2316600 A and W09944548 A1.

In view of the above, it is the object of the present invention that of suggesting an improved device permitting to the user to maintain the band in a grasping position in order to permit withdrawal of the same device after when the stocking has been put on, thus permitting a much easier application of the same.

It is therefore specific object of the present invention an auxiliary device for wearing an elastic stocking comprising a first piece of tissue, comprised of slippery material; and a second piece of tissue, comprised of slippery material, coupled along a first portion of its edge with a corresponding first portion of the edge of said first piece of tissue, so as to realise a funnel shaped pocket, closed on its bottom, said first and second pieces of tissue respectively comprising a first and a second guide slot, placed in correspondence of said pocket bottom, a band having a first end fixed in correspondence of the free portion of said edge of said first piece of tissue and a second end fixed in correspondence of the free portion of said edge of the second piece of tissue, said band passing through said first and second guide slots and having such a length to realise a ring in correspondence of said slots, said ring being usable for grasping when said device and said elastic stocking are put on, for pulling, folding and sliding each other said first and second pieces of tissue, thus easing extraction of the same device on said stocking; and means for coupling two points of the same band, suitable to prevent that the portion of the band realising said ring does not enter under the elastic stocking while it is put on said device, wherein said coupling means are removable.

Still according to the invention, said coupling means can comprise a snapping coupling system, such as a snapping button and/or a Velcro^{®} type coupling system.

Still according to the invention, said first element can be comprised of cartene (or of high density polyethylene) and/or of nylon and/or of tissue and/or of synthetic thermoplastic film and/or tetrafluoroethylene, polytetrafluoroethylene and/or fibreglass, while said second element can be comprised of cartene (or high density polyethylene) and/or nylon and/or tissue and/or synthetic thermoplastic film and/or tetrafluoroethylene, polytetrafluoroethylene and/or fibreglass.

The present invention will be now described, for illustrative but not limitative purposes, according to its preferred embodiments, with particular reference to the figures of the enclosed drawings, wherein:
figure 1 shows a top plan view of a first embodiment of the auxiliary device for wearing a therapeutic stocking according to the present invention;
figure 2 shows a bottom plan view of the device of figure 1;
figure 3 shows a perspective view of the device according to figure 1;
figures 4, 5 and 6 show particulars of the coupling means of the device according to figure 1;
figure 7 shows a lateral view of device of figure 1 put on a user;
figure 8 shows a lateral view of device of figure 1 put on a user, on which an elastic stocking is put on;
figure 9 shows a lateral view of device of figure 1 put on a user, on which an elastic stocking is completely put on;
figure 11 shows a bottom plan view of device according to figure 1 with a piece of fabric folded on itself;

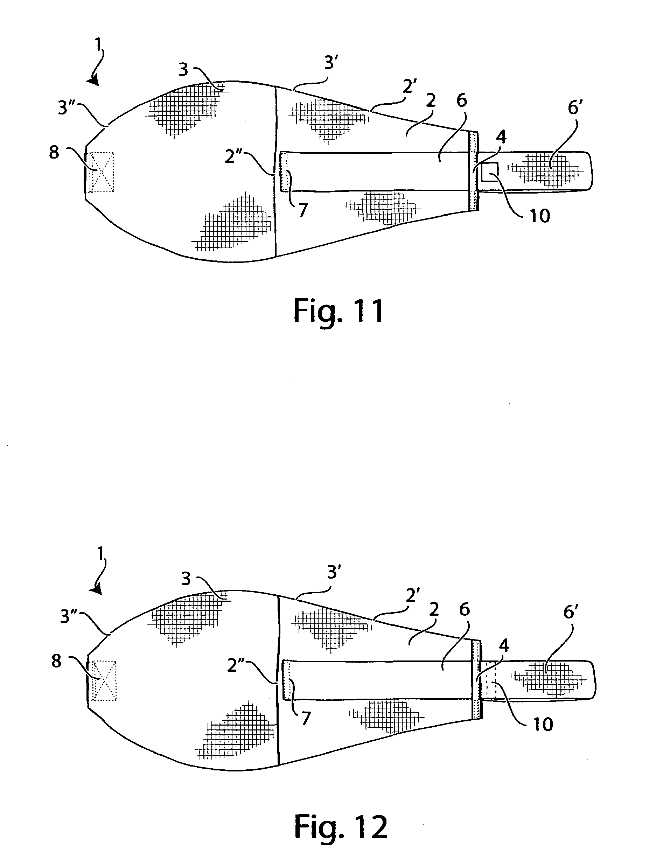

figure 11 shows a second embodiment of the auxiliary device for wearing a therapeutic stocking according to the present invention; and
figure 12 shows a third embodiment of the auxiliary device for wearing a therapeutic stocking according to the present invention.

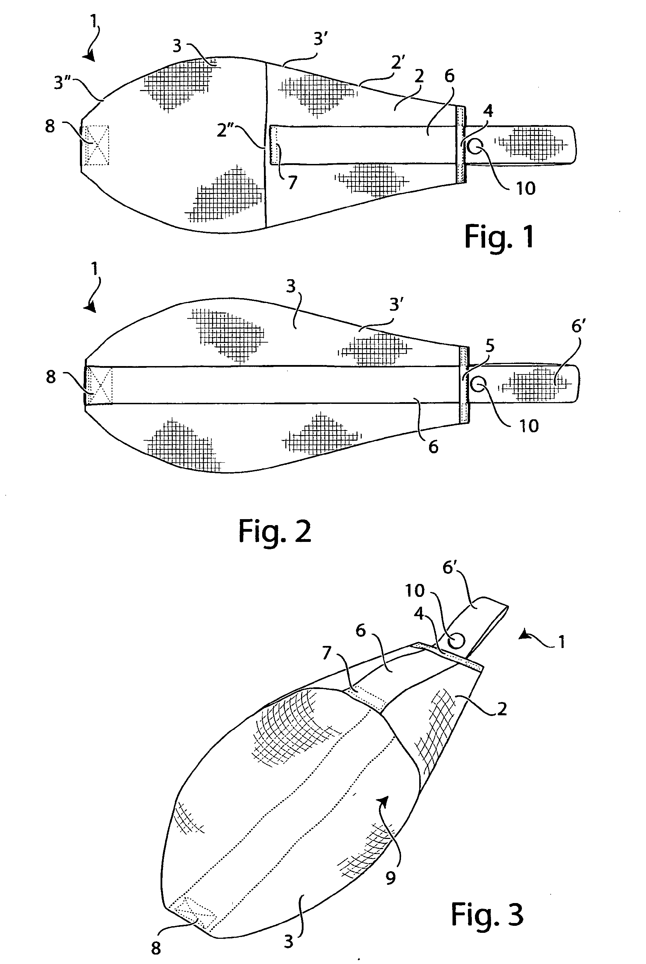

Making reference to figures 1, 2 and 3, it is possible observing a first embodiment of the auxiliary device 1 for wearing a therapeutic stocking according to the invention.

It is noted that device 1 includes a first piece of tissue 2, with its edge comprised of a first 2' and a second 2" portion, and a second piece of tissue 3, having its edge comprised of a first 3' and a second 3" portion.

Said first piece of tissue 2 is stitched along said first portion 2' of the edge with said second piece of tissue 3 along said first portion 3' of the edge.

From figure 3 it is noted that said first and second pieces of tissues 2, 3 are stitched along relevant portions 2', 3' of the edge, realising a kind of pocket 9 or division, within which, in an operative configuration, it can be introduced the limb on which the elastic stocking (not shown in the figure) must be put on.

Said pocket 9 has a closed funnel shape with its bottom stitched.

Said first and second pieces of fabric 2, 3 comprise two guide slots, respectively indicated by reference numbers 4 and 5. Said guide slots 4 and 5 are juxtaposed and provided in correspondence of the bottom of the pocket 9, on its lateral edges 2', 3'. They are also separated by a separation tissue edge.

Device 1 is also provided with a band 6, with a first end 7 stitched on said first piece of fabric 2, in correspondence of the free portion 2" of its edge. Instead, second end 8 is stitched on said second piece of fabric 3 in correspondence of the free portion 3" of its edge.

Said band 6 passes through both said guide slots 4, 5. Length of said band 6 is such to realise with its exceeding part, a kind of handle 6' in correspondence of said guide slots 4, 5.

Coupling means 10 are provided on said band, for coupling two portions or points of said band6. Said coupling means 10 are preferably provided, when the device is extended, close to said guide slots 4 and 5.

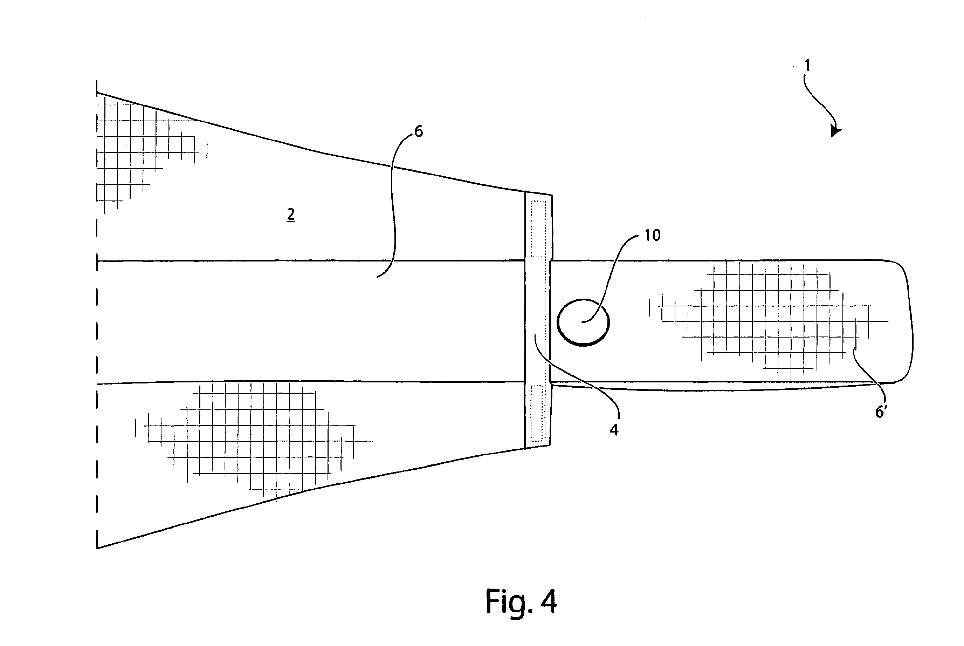

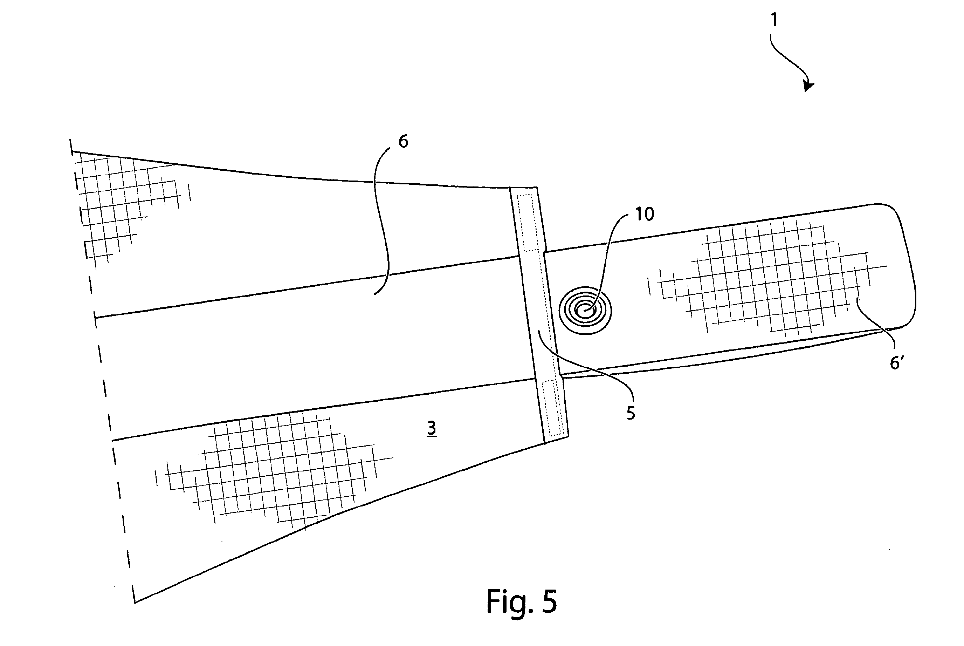

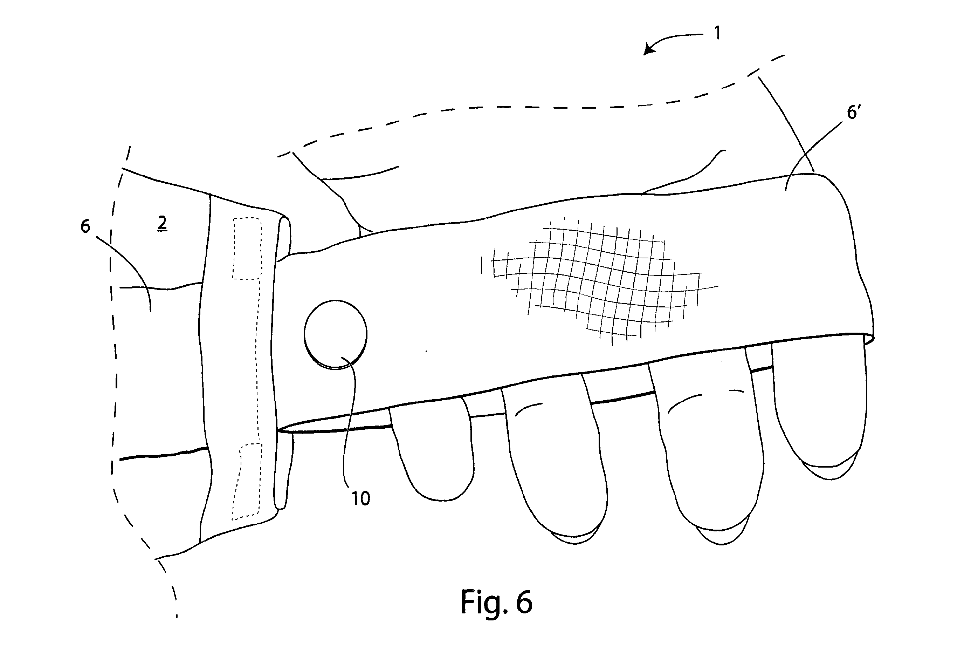

As it can be better observed from figures 4, 5 and 6, in the present embodiment said coupling means 10 are realised by a snapping system (e.g. a snapping button), comprised of two parts that can be coupled and separated each other, each part being fixed, e.g. by knitting or glue, to said band 6.

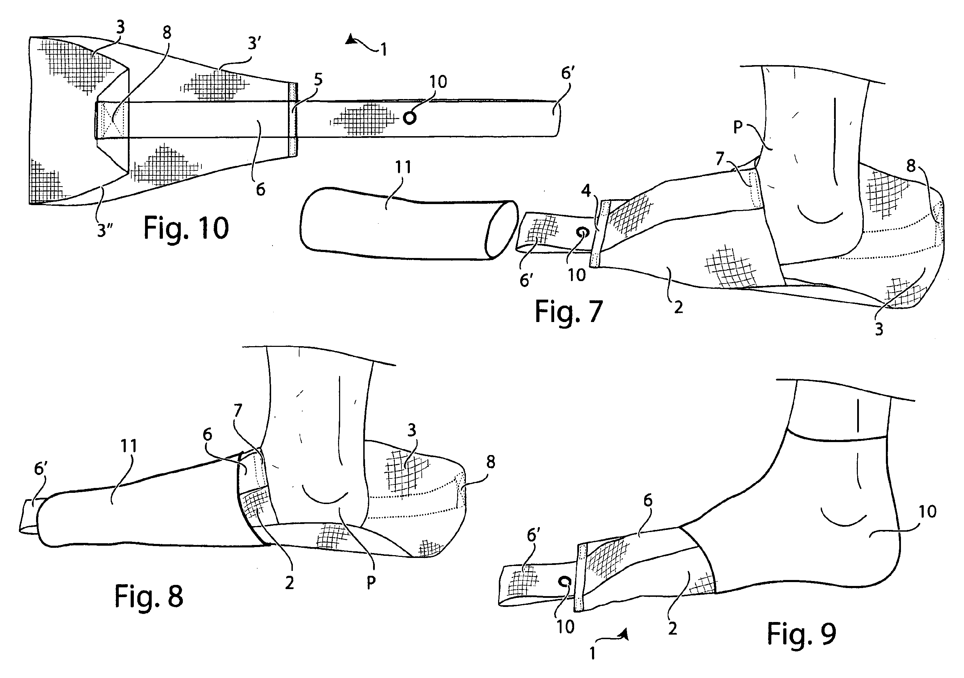

Figures 7, 8 and 9 show the operation of device 1 for easing the work of a user to put on an elastic stocking, said stocking being indicated in the figures by reference number 11.

Device 1 is shown in its embodiment for putting on an elastic stocking on a foot or on a leg, but the following specification is valid also for putting on elastic stockings 11 on other limbs, suitably varying dimensions of the device 1 and of the elastic stocking 11.

In order to easily put on the stocking, the user will have to close said coupling means 10, and then to introduce the foot P within the pocket 9 realised between said first and second pieces 2, 3 of fabric.

When the device 1 is put on by a user, it is easily possible putting on the elastic stocking 11.

When elastic stocking 11 is put on, the same can drag band 6, making it sliding up to the point where coupling means 10 will prevent its sliding, by the blocking action exerted by the tissue edge separating slots 4 and 5,

Now, once put on the elastic stocking 11, being the latter a tubular stocking, the user can easily grasp the band 6 portion remains outside said slots 4 and 5, thus realising a grasping element 6', which is kept outside said slots 4 and 5 just thanks to said coupling means 10.

Thus, then, to remove said device 1, now between said elastic stocking 11 and the limb, i.e. user foot P or leg, it is sufficient pulling the grasping piece of tissue 6' of the band 6, that will make at the same time a traction on both portions 2", 3" of the edges of said first and second pieces of tissue 2, 3, that, as it can be observed, are, the first one in correspondence of the feet back, and the second one under the feet P sole.

Both tissue pieces, after traction of the band 6, are folded, thus sliding and making removal of device 1 easier. This effect is particularly evidenced in figure 10 for the second piece 3 of fabric, showing that it is folded due to the traction by the band 6.

Device 1 is preferably comprised of low friction synthetic material. Anyway, said device 1 can be also comprised of different materials for each piece of tissue 2, 3, among which nylon, synthetic thermoplastic material film or tissue, tetrafluoroethylene, poli-tetrafluoroethylene, glass wool, cartene (or high density polyethylene).

Figures 11 and 12 show two further embodiments of the present invention. Particularly, figure 11 shows that coupling means are comprised of Velcro^{®}. Instead, embodiment of figure 12 shows that coupling means can be fix, in this case being comprised of a simple stitching.

An advantage of the present invention is that of preventing that, during insertion of stocking, band grasping can be inserted under the elastic stocking, thus making easy withdrawal of the auxiliary device from said stocking when put on; thus, present invention makes it easier application of device for putting on a therapeutic stocking.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Auxiliary device (1) for wearing an elastic stocking (11) comprising
a first piece of tissue (2), comprised of slippery material; and
a second piece of tissue (3), comprised of slippery material, coupled along a first portion (3') of its edge with a corresponding first portion (2') of the edge of said first piece of tissue (2), so as to realize an funnel shaped pocket (9), closed on its bottom;
said first (2) and second (3) pieces of tissue respectively comprising a first (4) and a second (5) guide slot, placed in correspondence of said pocket (9) bottom; and
a band (6) having a first end (7) fixed in correspondence of the free portion (2") of said edge of said first piece of tissue (2) and a second end (8) fixed in correspondence of the free portion (3") of said edge of the second piece of tissue (3), said band (6) passing through said first and second guide slots (4, 5) and having such a length to realize a ring (6') in correspondence of said slots (4, 5), said ring (6') being usable for grasping when said device (1) and said elastic stocking (11) are put on, for pulling, folding and sliding each other said first (2) and second (3) pieces of tissue, thus easing extraction of the same device (1) on said stocking (11) and means (10) for coupling two points of the same band (6), suitable to prevent that the portion of the band (6) realizing said ring (6') does not enter under the elastic stocking (11) while it is put on said device (1),
said device (1) being **characterized in that** said coupling means (10) are removable.

2. Device (1) according to claim 1, **characterized in that** said coupling means (10) comprise a snapping coupling system, such as a snapping button and/or a Velcro^{®} type coupling system.

3. Device (1) according to anyone of the preceding claims, **characterized in that** said first (2) and said second (3) tissue pieces are joined each other by stitching.

4. Device (1) according to anyone of the preceding claims, **characterized in that** said second piece of tissue (3) has a surface larger than said first piece of tissue (2).

5. Device (1) according to anyone of the preceding claims, **characterized in that** said first piece (2) is comprised of high density polyethylene and/or of nylon and/or of tissue and/or of synthetic thermoplastic film and/or tetrafluoroethylene, polytetrafluoroethylene and/or fibreglass.

6. Device (1) according to anyone of the preceding claims, **characterized in that** said second piece (3) is comprised of high density polyethylene and/or nylon and/or tissue and/or synthetic thermoplastic film and/or tetrafluoroethylene, polytetrafluoroethylene and/or fibreglass.

## Patentansprüche

1. Hilfsvorrichtung (1) zum Tragen eines elastischen Strumpfes (11), umfassend
ein erstes Stück Stoff (2), umfassend ein glattes Material; und
ein zweites Stück Stoff (3), umfassend ein glattes Material, welches entlang eines ersten Bereichs (3') seiner Kante mit einem entsprechenden ersten Bereich (2') der Kante des ersten Stücks Stoff (2) verbunden ist, um so ein trichterförmiges Fach (9) zu realisieren, welches an seinem Boden geschlossen ist; wobei
die ersten (2) und zweiten (3) Stücke Stoff entsprechend einen ersten (4) und einen zweiten (5) Führungsspalt aufweisen, der entsprechend dem Boden des Fachs (9) angeordnet ist; und
ein Band (6), das ein erstes Ende (7), das entsprechend dem freien Bereich (2") der Kante des ersten Stücks Stoffs (2) befestigt ist, und ein zweites Ende (8) aufweist, das entsprechend dem freien Bereich (3") der Kante des zweiten Stücks Stoff (3) befestigt ist, wobei das Band (6) durch die ersten und zweiten Führungsspalte (4, 5) hindurchtritt und eine derartige Länge aufweist, um einen Ring (6') entsprechend den Spalten (4, 5) zu realisieren, wobei der Ring (6') zum Greifen verwendet werden kann, wenn die Vorrichtung (1) und der elastische Strumpf (11) angezogen werden, zum Ziehen, Falten und Gleiten von jedem der ersten (2) und zweiten (3) Stücke Stoff, so ein Herausziehen derselben Vorrichtung (1) auf dem Strumpf (11) erleichternd, und Mittel (10) zur Verbindung von zwei Punkten des selben Bandes (6), geeignet zu verhindern, dass der Bereich des Bandes (6), welcher den Ring (6') realisiert, nicht unter den elastischen Strumpf (11) eintritt, während er auf die Vorrichtung (1) gezogen wird, wobei
die Vorrichtung (1) **dadurch gekennzeichnet ist, dass** die Verbindungsmittel (10) entfernbar sind.

2. Vorrichtung (1) gemäß Anspruch 1, **dadurch gekennzeichnet, dass** die Verbindungsmittel (10) ein Einschnappverbindungssystem, wie einen Einschnappknopf, und/oder ein Verbindungssystem vom Klettverschluss-Typ umfassen.

3. Vorrichtung (1) gemäß einem der vorhergehenden Ansprüche, **dadurch gekennzeichnet, dass** die ersten (2) und zweiten (3) Stücke Stoff miteinander durch eine Naht verbunden sind.

4. Vorrichtung (1) gemäß einem der vorhergehenden Ansprüche, **dadurch gekennzeichnet, dass** das zweite Stück Stoff (3) eine Fläche aufweist, die größer als die des ersten Stücks Stoff (2) ist.

5. Vorrichtung (1) gemäß einem der vorhergehenden Ansprüche, **dadurch gekennzeichnet, dass** das erste Stück Stoff (2) hochdichtes Polyethylen und/oder Nylon und/oder synthetischen thermoplastischen Film und/oder Tetrafluorethylen, Polytetrafluorethylen und/oder Glasfasern umfasst.

6. Vorrichtung (1) gemäß einem der vorhergehenden Ansprüche, **dadurch gekennzeichnet, dass** das zweite Stück Stoff (3) hochdichtes Polyethylen und/oder Nylon und/oder synthetischen thermoplastischen Film und/oder Tetrafluorethylen, Polytetrafluorethylen und/oder Glasfasern umfasst.

## Revendications

1. Dispositif auxiliaire (1) pour le port d'un bas élastique (11), comprenant :
une première pièce de tissu (2), constituée par un matériau glissant ; et
une deuxième pièce de tissu (3), constituée par un matériau glissant, couplée le long d'une première partie (3') de son bord à une première partie correspondante (2') du bord de ladite première pièce de tissu (2), de façon à réaliser une poche en forme d'entonnoir (9), fermée en son fond ;
lesdites première (2) et deuxième (3) pièces de tissu comprenant respectivement une première (4) et une deuxième (5) fentes de guidage, situées en correspondance avec le fond de ladite poche (9) ; et
une bande (6) comportant une première extrémité (7) fixée en correspondance avec la partie libre (2") dudit bord de ladite première pièce de tissu (2) et une deuxième extrémité (8) fixée en correspondance avec la partie libre (3") dudit bord de la deuxième pièce de tissu (3), ladite bande (6) traversant lesdites première et deuxième fentes de guidage (4, 5) et ayant une longueur telle qu'elle réalise un anneau (6') en correspondance avec lesdites fentes (4, 5), ledit anneau (6') pouvant être utilisé pour la saisie lorsque ledit dispositif (1) et ledit bas élastique (11) sont mis en place, pour tirer, plier et faire glisser l'une contre l'autre lesdites première (2) et deuxième (3) pièces de tissu, de façon à faciliter ainsi l'extraction de ce même dispositif (1) sur ledit bas (11), et des moyens (10) pour le couplage de deux points de cette même bande (6), appropriés pour empêcher que la partie de la bande (6) réalisant ledit anneau (6') n'entre pas sous le bas élastique (11) tandis qu'il est mis sur ledit dispositif (1),
ledit dispositif (1) étant **caractérisé en ce que** lesdits moyens de couplage (10) sont amovibles.

2. Dispositif (1) selon la revendication 1, **caractérisé en ce que** lesdits moyens de couplage (10) comprennent un système de couplage à encliquetage, tel qu'un bouton à encliquetage, et/ou un système de couplage du type Velcro^{®}.

3. Dispositif (1) selon l'une quelconque des revendications précédentes, **caractérisé en ce que** ladite première (2) et ladite deuxième (3) pièces de tissu sont réunies l'une à l'autre par couture.

4. Dispositif (1) selon l'une quelconque des revendications précédentes, **caractérisé en ce que** ladite deuxième pièce de tissu (3) a une surface supérieure à celle de ladite première pièce de tissu (2).

5. Dispositif (1) selon l'une quelconque des revendications précédentes, **caractérisé en ce que** ladite première pièce (2) est constituée par du polyéthylène haute densité et/ou du Nylon et/ou du tissu et/ou un film thermoplastique synthétique et/ou du tétrafluoroéthylène, du polytétrafluoroéthylène et/ou de la fibre de verre.

6. Dispositif (1) selon l'une quelconque des revendications précédentes, **caractérisé en ce que** ladite deuxième pièce (3) est constituée par du polyéthylène haute densité et/ou du Nylon et/ou du tissu et/ou un film thermoplastique synthétique et/ou du tétrafluoroéthylène, du polytétrafluoroéthylène et/ou de la fibre de verre.
